# FASCICULE DE BREVET EUROPEEN

(11) **EP 1 933 806 B1**
(45) Date de publication et mention de la délivrance du brevet: **23.08.2017**
(21) Numéro de dépôt: 06820269.6
(22) Date de dépôt: 09.10.2006
(51) Int. Cl.: A61K 8/92

(54) **EXCIPIENT COMPRENANT UN DERIVE DE CIRES POUR LA FABRICATION DE FORMULATIONS TOPIQUES COSMETIQUES**
STÜTZE MIT EINEM WACHSDERIVAT ZUR HERSTELLUNG VON TOPISCHEN KOSMETISCHEN FORMULIERUNGEN
SUPPORT COMPRISING A WAX DERIVATIVE FOR MAKING TOPICAL COSMETIC FORMULATIONS

(30) Priorité: 12.10.2005 FR 0553101
(43) Date de publication de la demande: 25.06.2008
(73) Titulaire: GATTEFOSSE SAS, 69804 Saint Priest (FR)
(72) Inventeur: RODIER, Jean-David, F-69100 Villeurbanne (FR); HUBICHE, Vincent, F-69720 Saint Laurent de Mure (FR); MAHLER, Bruno, F-69006 Lyon (FR)
(74) Mandataire: Cabinet Laurent & Charras
(86) Numéro de dépôt international: PCT/FR2006/051007
(87) Numéro de publication internationale: WO 2007/042723

(56) Documents cités:
- EP-B- 1 286 679
- US-A- 5 387 579
- US-A- 5 660 865
- US-A- 5 874 092
- US-B2- 6 706 768

## Description

L'invention a pour objet un procédé de préparation d'un excipient comprenant un dérivé de cires, pour la fabrication notamment de formulations topiques cosmétiques. En pratique, cet excipient peut être utilisé en cosmétique et pharmacie, en particulier pour son effet émulsionnant suivant les cas E/H ou H/E, de même que pour son effet émollient. L'invention est plus particulièrement décrite par la suite en relation avec le domaine cosmétique bien qu'elle puisse être mise en oeuvre, comme déjà dit, en pharmacie.

Les cires sont largement utilisées en cosmétique, en particulier comme agent durcissant dans les rouges à lèvres ou comme agent épaississant dans certaines émulsions. Leur composition est particulièrement complexe. Elles ont la particularité commune de contenir un mélange de monoesters d'acides et d'alcools gras à très longues chaînes.

On distingue les cires solides et les cires liquides.

Les cires solides sont des cires, dont le point de fusion se situe entre 50 et 90°C. Elles correspondent à des mélanges comprenant essentiellement des monoesters de formule R¹-C(O)-O-R², où le groupement R¹-C(O)- correspond à la chaîne carbonée de l'acide gras, ledit acide étant le plus souvent linéaire et saturé et présentant un nombre de carbone supérieur ou égal à 20. On inclut donc l'acide comprenant au moins 20 atomes de carbone, c'est-à-dire l'acide eicosanoïque (ou arachidique), les acides en C22, docosanoïque (ou béhénique), C24, tétracosanoïque (ou lignocérique), C26, hexacosanoïque (ou cérotique) jusqu'à C34. En fonction de l'origine de la cire, le mélange de monoesters peut également contenir une certaine proportion d'esters d'hydroxyacides tels que l'acide hydroxypalmitique (C16), ou hydroxystéarique. C'est par exemple le cas de la cire d'abeille. Le groupement R² correspond à la chaîne hydrocarbonée de l'alcool, ledit alcool étant le plus souvent linéaire saturé et présentant un nombre de carbone supérieur ou égal à 20. Il peut donc s'agir de l'eicosanol en C20, du docosanol en C22, du tetracosanol en C24 et ainsi jusqu'à C34. En fonction de l'origine de la cire, le mélange de monoesters peut également contenir une certaine proportion d'esters de diols. C'est par exemple le cas de la cire de carnauba.

La cire d'abeille, la cire de carnauba, la cire de candelilla, la cire de riz et la cire de canne à sucre sont des exemples de cires solides, naturelles.

Les cires liquides sont des mélanges de monoesters d'acides et d'alcool gras à longue chaîne se présentant dans un état liquide à température ambiante soit une température comprise entre 15°C et 25°C environ. Ces monoesters ont la formule suivante R³-C(O)-O-R⁴, où le groupement R³-C(O) correspond à la chaîne carbonée de l'acide gras. Cette chaîne peut être linéaire et monoinsaturée. Elle comprend un nombre de carbone supérieur ou égal à 18. On peut citer l'acide oléique (C18 :1), gadoléique (C20 :1), érucique (C22 :1) jusqu'à l'acide hexacosénoique (C26 :1) pour les acides insaturés. Le groupement R³-C(O) peut aussi être constitué par les acides ramifiés et saturés présentant un nombre de carbone supérieur à C18, appelés aussi acides de Guerbet. Le groupement R⁴-O- peut être constitué par les alcools gras linéaires monoinsaturés avec un nombre de carbone supérieur ou égal à 18. On peut donc citer l'octadécénol en C18, l'eicosénol en C20, le docosénol en C22 jusqu'en C26, hexacosénol. La chaîne carbonée de l'alcool peut également être ramifiée et saturée et comprendre un nombre de carbone supérieur ou égal à C18. De tels alcools sont appelés aussi alcools de Guerbet. Une cire liquide connue à ce jour est la cire de jojoba communément appelée huile de jojoba, la nature liquide étant due à la présence de chaînes monoinsaturées.

Le document US 5 660 865 décrit l'utilisation en cosmétique d'un mélange de cires solides et de cires liquides sans adjonction de polyol.

Le document US 6 630 134 B1 décrit l'utilisation cosmétique d'esters de cire solide d'abeille ou de candelilla ou de carnauba avec un alcool de guerbet. Les trois produits obtenus à partir des trois cires sont utilisés dans des gels pour les cheveux.

Le document US 6 432 428 B1 décrit l'utilisation en cosmétique, notamment comme émollient, de dérivés de cire de jojoba et de jojoba hydrogénée obtenus par alcoolyse par l'alcool isopropylique.

Le document EP 795 317 B1 décrit une composition cosmétique destinée au traitement de la peau ou des cheveux comprenant une association de cires constituée de cire molle telle que cire de pomme et cire d'orange et d'une cire liquide, en l'espèce la cire de jojoba.

Le document US 6 258 965 décrit le produit de réaction entre une cire et de l'huile de graines de reine des prés (Limnanthes Alba), appelée aussi huile de meadowfoam, correspondant à un triglycéride contenant des acides gras monoinsaturés à longues chaînes en C20 et C22. En pratique, il se produit une réaction d'interestérification entre les acides gras du triglycérides et les alcools gras constitutifs de la cire.

Le document US 6 706 768 B2 décrit une composition obtenue par saponification, puis alkoxylation d'un mélange de cire de jojoba hydrogénée et non hydrogénée en l'absence de polyol. Le mélange obtenu contient des éthers.

A la connaissance du Demandeur, il existe également sur le marché, des mélanges de cires solide et liquide modifiées séparément avec des polyols. On peut citer par exemple, Jojoba wax PEG-80 Esters (Florasolv^{®} de Floratech), PEG-8 Beeswax (produit de la réaction entre cire d'abeille et PEG) (Apifil^{®} de Gattefossé) mentionné dans le document EP 1 286 679 B1, Bis-PEG-12 Dimethicone Beeswax (Siliconyl Beeswax de Koster Keunen), Polyglyceryl-3 Beeswax (Jan Dekker et Koster Keunen) mentionné dans le document US-A-5 387 579.

Le Demandeur a constaté que de manière tout à fait surprenante, le produit de réaction simultanée entre une cire solide, une cire liquide et un polyol, présentait une dureté plus faible que le produit issu du mélange des deux produits réactionnels ci-avant décrits respectivement une cire solide modifiée par un polyol et une cire liquide modifiée par ce même polyol. Ce comportement différent pourrait provenir, selon le Demandeur, de la présence d'un nouveau dérivé de polyol n'existant pas lorsque les produits sont mélangés séparément, ce dérivé de polyol correspondant à une structure dans laquelle toute ou partie des fonctions OH du polyol sont estérifiées avec les acides gras linéaires saturés de la cire solide et les acides gras linéaires insaturés ou ramifiés saturés de la cire liquide. De plus, par cette réaction de transestérification, on obtient une redistribution aléatoire des acides et alcools composant les cires. Le dérivé de cires contient donc en fin de réaction des esters d'acides linéaires mono insaturés ou ramifiés saturés et d'alcools linéaires saturés, des esters d'acides linéaires saturés et d'alcools linéaires mono insaturés ou ramifiés saturés, des alcools libres linéaires saturés, des alcools libres linéaires monoinsaturés et/ou ramifiés saturés et les esters de polyol précédemment décrits, en plus des constituants de départ qui n'auraient pas réagi.

En d'autres termes, l'invention a pour objet un procédé de préparation d'un excipient, en particulier pour la fabrication de formulations cosmétiques topiques. Cet excipient comprend un mélange contenant essentiellement:
- des esters de polyol avec des acides linéaires saturés et des acides linéaires insaturés et/ou ramifiés saturés,
- des esters d'acides monoinsaturés linéaires ou saturés ramifiés avec des alcools linéaires saturés,
- des esters d'acides linéaires saturés avec des alcools monoinsaturés linéaires ou saturés ramifiés,
- des alcools libres linéaires saturés et/ou linéaires monoinsaturés et/ou ramifiés saturés,
- des esters d'acides linéaires saturés avec des alcools linéaires saturés
- des esters d'acides linéaires monoinsaturés ou ramifiés saturés avec des alcools linéaires monoinsaturés ou ramifiés saturés,
les acides et alcools linéaires saturés présentant au moins 20 atomes de carbone et les acides et alcools monoinsaturés ou ramifiés saturés présentant au moins 18 atomes de carbone.

Ce mélange est un dérivé de cires obtenu par réaction entre elles d'au moins une de cire solide et au moins une cire liquide en présence d'au moins un polyol et d'au moins un catalyseur. Comme déjà dit, il se produit une réaction de transestérification entre les différentes entités chimiques conduisant au produit ci-dessus.

La présente invention concerne donc un procédé de préparation d'un excipient par réaction de transestérification simultanée entre elles d'au moins une cire solide et d'au moins une cire liquide en présence d'au moins un polyol et d'un catalyseur.

En pratique, la cire solide est une cire naturelle avantageusement choisie dans le groupe comprenant la cire de carnauba, la cire de candelilla, la cire de son de riz, la cire de tournesol, la cire de canne à sucre, la cire d'ouricury, la cire d'abeille, la cire de Shellac.

De même, la cire liquide est une cire naturelle, en pratique de la cire de jojoba. Cette cire comprend notamment des esters d'acides gras insaturés C18:1 (minoritaire), C20:1 et C22:1 (majoritaires avec C20:1 > C22:1), avec des alcools gras insaturés C20:1 et C22:1 puis C24:1.

Dans un mode de réalisation particulier, le mélange est obtenu en faisant réagir des acides et alcools linéaires saturés avec des acides et alcools linéaires monoinsaturés et/ou ramifiés saturés, et des polyols, en présence de catalyseur, les acides et alcools linéaires saturés présentant au moins 20 atomes de carbone et les acides et alcools linéaires monoinsaturés ou ramifiés saturés présentant au moins 18 atomes de carbone. Il se produit alors une réaction d'estérification.

Selon une autre caractéristique, le polyol est choisi dans le groupe comprenant l'éthylène glycol, le diéthylène glycol, le triéthylène glycol, le 2-méthyl propanediol, le propylène glycol, le butylène glycol, le néopentyl glycol, hexylène glycol, octylène glycol, polyéthylène glycol, polypropylène glycol, triméthylol propane, sorbitol, érythritol, pentaerythritol, dipentaerythritol, glycérol, diglycérol, polyglycérol.

Lorsque l'excipient est préparé à partir de cire, la présence de polyol dans la réaction confère au dérivé de cires une certaine hydrophilie et donc des propriétés tensioactives. L'incorporation d'un polyol augmente la teneur en OH libres dans le produit final. Le comportement tensioactif H/E ou E/H du dérivé variera en fonction de la nature et de la proportion de polyol mis en oeuvre.

Un dérivé de cires obtenu à partir principalement de cire liquide, de cire solide et de polyglycérol possède un comportement d'émulsionnant eau dans huile ainsi qu'une capacité à absorber de l'eau comme le fait naturellement la lanoline.

En pratique, la proportion de polyol estérifié représente entre 0.5 et 50% en poids du mélange, la proportion d'acides gras estérifiés représente entre 20 et 60% en poids du mélange et la proportion d'alcools gras estérifiés entre 20 et 60% en poids du mélange.

Les transformations moléculaires intervenant au cours de la réaction de transestérification sont obtenues en présence de catalyseurs. Les catalyseurs préférés sont les hydroxydes ou alkoxydes alcalins ou alcalino terreux, tels que la soude et la potasse, en solution alcoolique, aqueuse ou sous forme solide, l'hydroxyde de calcium, les carbonates de potassium ou de sodium, les catalyseurs à base d'étain ou de titane.

Dans un mode de réalisation préféré, le dérivé de cire est obtenu par réaction de cire de jojoba, de cire de candelilla, de cire de riz, en présence de polyglycérol.

En pratique, la réaction est conduite à une température comprise entre 100°C et 220°C, avantageusement entre 150 et 200°C.

En fonction des propriétés recherchées de l'excipient, la proportion massique cire liquide/cire solide, de même que la proportion massique cires/polyol varie.

En pratique, le rapport massique cire liquide/cire solide varie entre 5/95 et 95/5, avantageusement entre 30/70 et 75/25. Le rapport massique cires /polyol varie quant à lui, entre 1/99 et 99/1, avantageusement entre 95/5 et 50/50.
L'excipient présente des propriétés émulsionnantes eau-dans-huile et huile-dans-eau et ce, en fonction du polyol choisi. L'utilisation par exemple, de polyglycérol permet d'obtenir un émulsionnant lipophile donnant des émulsions eau-dans-huile, eu égard à la capacité du dérivé obtenu à absorber l'eau en grande quantité. Au contraire, l'utilisation par exemple de PEG permet d'obtenir un agent émulsionnant hydrophile donnant des émulsions huile-dans-eau.

L'excipient précédemment décrit peut être utilisé comme émulsionnant E/H ou H/E.

Dans un mode de réalisation particulier, l'excipient, lorsqu'il est utilisé comme agent émulsionnant, contient au moins le dérivé de cires précédemment décrit et jusqu'à avantageusement trois, des constituants choisis dans le groupe des stearoyl lactylate de sodium, alcool cétostéarylique, stéarate de glycérol.

Avantageusement, l'excipient est un système émulsionnant constitué d'un dérivé de cires issu de la réaction entre la cire de jojoba, cire de son de riz, cire de candelilla, en présence de polyglycérol et des quatre constituants précédents (stéaroyle lactylate de sodium 4-50%, alcool cétostéarylique 4-50%, stearate de glycérol 4-50%, dérivé de cires 4-50% en poids par rapport au poids d'excipient).

L'excipient précédemment décrit peut être utilisé dans une composition cosmétique.

Lorsque l'excipient consiste en un dérivé de cire précédemment décrit, le dérivé de cire représente entre 0,01 et 99% en poids, de préférence entre 0,1 et 10% en poids de la composition cosmétique.

L'excipient est en général appliqué par voie topique. Il présente en outre des qualités sensorielles de douceur à l'application et au final particulièrement appréciées.

L'excipient en général, et les dérivés de cire en particulier, peuvent être formulés sous toutes les formes galéniques normalement utilisées pour une application topique sur la peau ou les cheveux, notamment au sein d'une composition cosmétique sous forme d'une émulsion huile-dans-eau ou eau-dans-huile ou multiple, d'une émulsion siliconée, d'une microémulsion ou nanoémulsion.

La composition peut être plus ou moins fluide et avoir l'aspect entre autre d'une crème blanche ou colorée, d'une pommade, d'un lait, d'une lotion, d'un sérum.

La composition peut contenir les adjuvants habituels dans les domaines cosmétique et dermatologique, tels que les matières grasses, les émulsionnants et co-émulsionnants, les gélifiants hydrophiles ou lipophiles, les actifs hydrophiles ou lipophiles, les conservateurs, les antioxydants, les solvants, les parfums, les charges, les filtres hydrophiles et lipophiles, les matières colorantes, les neutralisants, les agents propénétrants, et les polymères.

Les quantités de ces différents adjuvants sont celles classiquement utilisées dans les domaines considérés, et par exemple de 0.01 à 30% du poids total de la composition. Ces adjuvants, selon leur nature, peuvent être introduits dans la phase grasse ou dans la phase aqueuse.

Comme matières grasses utilisables dans une composition cosmétique, on peut utiliser les huiles minérales, les huiles d'origine animale (lanoline), les huiles végétales, les huiles de synthèse (isopropyl myristate, octyldodecyl, isostearyl isostearate, decyl oleate, isopropyl palmitate), les huiles siliconées (cyclomethicone, dimethicone) et les huiles fluorées. On peut utiliser comme matières grasses des alcools gras, des acides gras, des cires et des gommes et en particulier les élastomères de silicone.

Comme émulsionnants et co-émulsionnants utilisables dans une composition cosmétique, on peut citer par exemple les esters de polyglycérols et d'acides gras, les esters de saccharose et d'acides gras, les esters de sorbitane et d'acides gras, les esters d'acides gras et de sorbitan polyoxyéthylénés, les éthers d'alcools gras et de PEG, les esters de glycérol et d'acides gras, les alkyl sulfates, les alkyl éther sulfates, les alkyl phosphates, les alkyl polyglucosides, les dimethicones copolyols, et les dérivés de l'acide citrique et de l'acide lactique tel que le Sodium Stearoyl Lactylate.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides tels que la gomme xanthane, la gomme guar, les gommes naturelles tels que la gomme de cellulose et ses dérivés, les amidons et leurs dérivés, les argiles et les copolymères d'acide 2-acrylamido-2-méthylpropane.

Comme gélifiants lipophiles, on peut citer les argiles modifiées comme les bentones, les sels métalliques d'acides gras, la silice et ses dérivés et l'éthylcellulose.

La composition cosmétique peut également contenir des actifs. Comme actifs, on peut utiliser notamment les dépigmentants, les émollients, les hydratants, les anti-séborrhéiques, les anti-inflammatoires, les anti-acnéiques, les agents kératolytiques et/ou desquamants, les agents anti-rides et tenseurs, les agents drainants, les agents anti-irritants, les agents apaisants, les amincissants tels que les bases xanthiques (caféine), les vitamines et leurs mélanges, les agents matifiants, les actifs anti-âge tel que le retinol, les agents anti-rides, et les huiles essentielles.

Comme conservateurs utilisables, on peut citer l'acide benzoïque, ses sels et ses esters ; l'acide sorbique et ses sels ; les parabens, leurs sels et esters ; le triclosan ; l'imidazolidinyl urée ; le phenoxyethanol ; la DMDM hydantoïne ; le diazolidinyl urée ; la chlorphenesin.

Comme antioxydants utilisables, on peut citer les agents chelatants tels que l'EDTA et ses sels.

Comme solvants utilisables, on peut citer l'eau, l'éthanol, la glycérine, le propylène glycol, le butylène glycol, le sorbitol.

Comme charges utilisables, on peut citer le talc, le kaolin, le mica, la serecite, le magnésium carbonate, l'aluminium silicate, le magnésium silicate, les poudres organiques telles que le nylon.

Comme filtres utilisables, on peut citer les filtres UVA et UVB classiquement utilisés tels que la benzophenone-3, le butyl methoxydibenzoyl methane, l'octocrylène, l'octyl methoxycinnamate, le 4-methylbenzylidene camphor, l'octyl salycylate, le tacephthalydene dicamphor sulfanic acid, et le drométrizole trisiloxane. On citera également les filtres physiques TiO2 et ZnO sous leurs formes micrométriques et nanométriques, enrobés ou non enrobés.

Comme matières colorantes utilisables, on peut citer les colorants lipophiles, les colorants hydrophiles, les pigments et les nacres habituellement utilisés dans les compositions cosmétiques ou dermatologiques, et leurs mélanges.

Comme neutralisants utilisables, on peut citer la soude, la triethanolamine, l'aminomethyl propanol, l'hydroxyde de potassium.

Comme agents propénétrants utilisables, on peut citer les alcools et glycols (éthanol, propylène glycol), l'éthoxydiglycol, les alcools et acides gras (acide oléique), les esters d'acides gras, le dimethyl isosorbide.

Cette composition peut être utilisée comme produit de soin, comme produit de nettoyage, et/ou comme produit de maquillage de la peau, comme produit de protection solaire, ou comme produit capillaire.

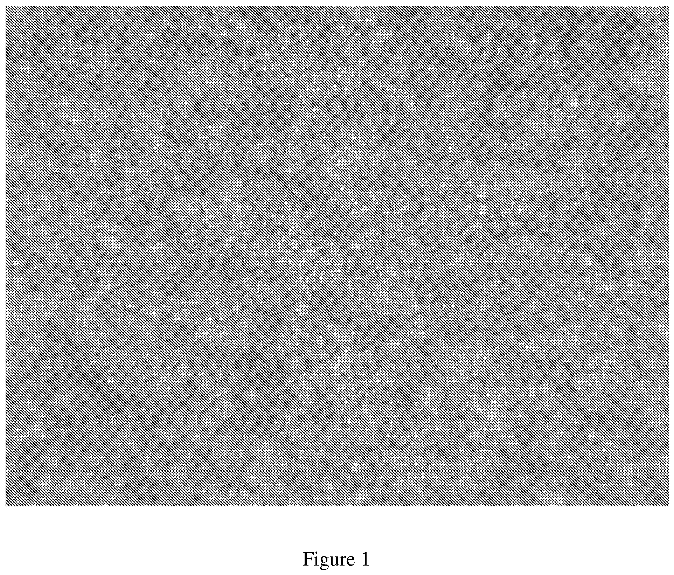

L'invention et les avantages qui en découlent ressortiront bien des exemples de réalisation suivants à l'appui de la figure annexée. La figure 1 est une vue microscopique d'une émulsion formée par un dérivé de cire obtenu selon l'invention.

### Exemple 1 : Obtention du dérivé de cires dans des proportions massiques de cire liquide/cire solide d'environ 70/30 et de cires/polyol de 85/15

**Composition du mélange réactionnel de départ**

| | |
|---|---|
| Cire de jojoba | 571 g |
| Cire de candelilla | 123 g |
| Cire de riz | 122 g |
| Triglycérol | 144 g |
| K2CO3 | 40 g |

Les produits sont introduits dans un réacteur agité de 2 litres. La réaction se déroule à 200°C, pendant 8 heures sous un inertage à l'azote. A la fin du palier de température, le catalyseur est neutralisé. Le dérivé de cires est récupéré puis filtré. La quantité de dérivé de cires récupérée est d'environ 780 g. Le produit obtenu est de couleur beige et d'une consistance molle.

Le produit possède la capacité à former des émulsions eau dans huile. Une vue microscopique de l'émulsion montre des gouttes d'eau dispersées dans la phase cireuse.

L'invention et les avantages qui en découlent ressortiront bien des exemples de réalisation suivants à l'appui de la figure1 qui est une photographie prise au microscope de l'émulsion de l'exemple 1.

### Exemple 2 : Obtention du dérivé de cires dans des proportions de cire liquide/cire solide de 50/50 et de cires/polyol de 90/10

| | |
|---|---|
| Cire de jojoba | 432 g |
| Cire de candelilla | 432 g |
| PEG 200 | 96 g |
| NaOH aqueuse | 40 g |

Les produits sont introduits dans un réacteur agité de 2 litres. La réaction se déroule à 200°C, pendant 8 heures sous un inertage à l'azote. A la fin du palier de température, le catalyseur est neutralisé par une quantité suffisante d'acide citrique. Le dérivé de cires est ensuite filtré. La quantité récupérée est d'environ 800 g. Le produit obtenu est beige clair et d'une consistance molle.

Le produit possède la capacité à former des émulsions huile dans eau.

### Exemple 3 : Obtention du dérivé de cires dans des proportions massiques de cire liquide/cire solide d'environ 60/40 et de cires/polyol de 80/20

**Composition du mélange réactionnel de départ**

| | |
|---|---|
| Cire de jojoba | 461 g |
| Cire de riz | 307 g |
| Propylène glycol | 192 g |
| K2CO3 | 40 g |

Les produits sont introduits dans un réacteur agité de 2 litres. La réaction se déroule à 150°C, pendant 8 heures sous un inertage à l'azote. A la fin du palier de température, le catalyseur est neutralisé par une quantité suffisante d'acide phosphorique. Le propylène glycol en excès est éliminé sous une pression de 5 mBar et le dérivé de cires finalement obtenu est ensuite filtré. La quantité de dérivé de cires récupérée est d'environ 680 g. Le produit obtenu est marron clair et d'une consistance molle.

Le produit épaissit les formules cosmétiques et peut être utilisé comme agent de consistance.

### Exemple 4 : Essais comparatifs de dureté, évaluation de la consistance du dérivé de cire

Dans cet exemple, on compare la dureté du dérivé de cire de l'exemple 1 avec celle du produit résultant du mélange des trois dérivés de chaque cire préparés séparément. Le mode opératoire appliqué pour obtenir les dérivés de cires est celui cité dans l'exemple 1.

On constate que le produit de réaction est beaucoup plus mou que le mélange. On souhaite mettre en avant cette caractéristique par la mesure de dureté traduite par la profondeur de pénétration d'un pointeau appliquant une force limite de 2N.

### Appareillages utilisés pour la mesure de dureté

- support TCD 200 de marque Chatillon
- dynamomètre Indelco DGGS, équipé d'un pointeau, limité à 2.5N

Principe : le pointeau va pénétrer l'échantillon. La force appliquée va donc augmenter. On mesure ensuite la profondeur de pénétration lorsque la force atteint 2N.

On obtient les résultats suivants :
Dérivé de cires : 4.4 mm
Mélange des dérivés : 2.4 mm

Le dérivé de cires est donc beaucoup plus mou que le mélange de chaque cire dérivée. Le dérivé de cires de l'exemple 1 ainsi que le mélange de chaque dérivé ont la même composition en chacune des cires (candelilla, riz et jojoba).

On note ainsi l'importance du dérivé de polyol contenant des chaînes acides monoinsaturés et saturés, ainsi que produit de réaction des cires entre elles, qui ramollissent le produit final.

### Exemple 5 : Exemples de formulation

**Crème de jour anti-rides**

| Ingrédients | Quantité (%) |
|---|---|
| **Dérivé de cires selon l'exemple 1** | **2.00** |
| Sodium Stearoyl Lactylate | 0.50 |
| Acide stéarique | 1.00 |
| Cyclopentasiloxane | 3.00 |
| Myristate d'Octyldodécyle | 2.00 |
| Alcool cétostéarylique | 1.00 |
| Stéarate de glycérol | 0.50 |
| Octyl Methoxycinnamate | 5.00 |
| Benzophenone-3 | 2.00 |
| Aluminium starch octenylsuccinate | 3.00 |
| Phenoxyethanol, Methylparaben, Butylparaben, Ethylparaben, Propylparaben | 1.00 |
| Amidon modifié | 1.50 |
| Gomme xanthane | 0.20 |
| EDTA disodique | 0.05 |
| Glycérine | 3.00 |
| Extrait liquide d'Acmella Oleacera | 2.00 |
| Ethanol | 3.00 |
| Acétate de tocophérol | 0.50 |
| Parfum | 0.40 |
| Eau | Qsp 100 |

**Lait corporel**

| Ingrédients | Quantité (%) |
|---|---|
| PEG-6 Stearate, Ceteth-20, Steareth-20 | 8.00 |
| Propylene Glycol Dipelargonate | 10.00 |
| Acide stéarique | 1.00 |
| **Dérivé de cires selon l**'**exemple 2** | **2.00** |
| Huile de noyaux | 3.00 |
| Dimethicone | 2.00 |
| Acétate de Tocopherol | 0.50 |
| Cyclomethicone | 3.00 |
| Phenoxyethanol, Methylparaben, Butylparaben, Ethylparaben, Propylparaben | 1.00 |
| Carbomer | 0.15 |
| Gomme xanthane | 0.30 |
| Glycérine | 3.00 |
| Sodium Hydroxide (10% solution) | 0.30 |
| Acide Ascorbique | 0.05 |
| Parfum | 0.40 |
| Eau | Qsp 100 |

**Emulsion H/E**

| Ingrédients | Quantité (%) |
|---|---|
| Phenoxyethanol, Methylparaben, Butylparaben, Ethylparaben, Propylparaben | 1.0 |
| Eau | Qsp 100 |
| Magnésium aluminium silicate | 1.5 |
| Glycerine | 3.0 |
| Gomme Xanthane | 0.1 |
| Polysorbate-60 | 0.9 |
| Glyceryl Stearate, PEG-100 Stearate | 2.1 |
| Alcool Cetylique | 2.6 |
| **Dérivé de cires selon l'exemple 3** | **1.5** |
| Huile de paraffine | 7.5 |
| Myristate d'Isopropyle | 7.5 |
| Ethoxydiglycol | 5.0 |
| Parfum | 0.2 |
| Triethanolamine | 0.3 |

**Emulsion E/H**

| Ingrédients | Quantité (%) |
|---|---|
| Glycérine | 3.0 |
| Propylene Glycol, Diazolidinyl Urea, Methylparaben, Propylparaben | 1.0 |
| Sulfate de Magnésium | 0.7 |
| Cetyl Dimethicone Copolyol | 2.5 |
| **Dérivé de cires selon l'exemple 1** | **3.0** |
| Isohexadecane | 3.5 |
| Caprylic/Capric Triglycéride | 5.8 |
| Dimethicone | 4.0 |
| Parfum | 0.1 |
| Eau | Qsp 100 |

**Emulsion multiple W/O/W**

| Ingrédients | Quantité (%) |
|---|---|
| PEG-30 Dipolyhydroxystearate | 2.40 |
| Isohexadecane | 9.00 |
| PPG-15 Stearyl Ether | 4.50 |
| **Dérivé de cires selon l**'**exemple 3** | **1.20** |
| Caprylic/Capric Triglycéride | 4.50 |
| Sulfate de magnesium | 0.82 |
| Propylene Glycol, Diazolidinyl Urea, Methylparaben, Propylparaben | 1.20 |
| Poloxamer 407 | 2.00 |
| Glycerine | 3.00 |
| Gomme Xanthane | 0.70 |
| Parfum | 0.20 |
| Eau | Qsp 100 |

**Crème solaire**

| Ingrédients | Quantité (%) |
|---|---|
| DEA Cetyl Phosphate | 2.0 |
| Glyceryl Stearate, PEG-100 Stearate | 4.0 |
| **Dérivé de cires selon l**'**exemple 2** | **2.0** |
| Octyl Methoxycinnamate | 7.0 |
| Butyl Methoxydibenzoylmethane | 2.0 |
| Benzophenone-3 | 1.0 |
| Titanium Dioxide | 3.0 |
| Cocoate de butylene glycol | 3.0 |
| Cyclomethicone | 2.0 |
| Acetate de Tocopherol | 0.5 |
| EDTA | 0.1 |
| Acrylates/C10-30 Alkyl Acrylates Crosspolymer | 0.2 |
| Gomme Xanthane | 0.3 |
| Phenoxyethanol, Methylparaben, Ethylparaben, Propylparaben, Isobutylparaben | 1.0 |
| Butylene Glycol | 3.0 |
| Hydroxide de Sodium (solution 10%) | 0.4 |
| Parfum | 0.3 |
| Eau | Qsp 100 |

**Fond de teint**

| Ingrédients | Quantité (%) |
|---|---|
| Glyceryl Stearate, Propylene Glycol Stearate, Glyceryl Isostearate, Propylene Glycol Isostearate, Oleth-25, Ceteth-25 | 5.00 |
| **Dérivé de cires selon l**'**exemple 3** | **4.00** |
| Glyceryl Dibehenate, Tribehenin, Glyceryl Behenate | 1.00 |
| Oleate d'ethoxydiglycol | 9.00 |
| Isostearate d'Isostearyle | 5.00 |
| Alcool Cetostearylique | 2.00 |
| Dimethicone | 5.00 |
| Acetate de Tocopherol | 0.50 |
| Phenoxyethanol, Methylparaben, Ethylparaben, Propylparaben, Isobutylparaben | 0.60 |
| Gomme Xanthane | 0.40 |
| Microcrystalline Cellulose, Cellulose Gum | 1.50 |
| Titanium Dioxide | 6.60 |
| Iron Oxides (Yellow pigment) | 1.55 |
| Iron Oxides (Red Pigment) | 0.43 |
| Iron Oxides (Black pigment) | 0.11 |
| Dimethicone, Dimethiconol | 3.00 |
| Eau | Qsp 100 |

## Revendications

1. Procédé de préparation d'un excipient par réaction de transestérification simultanée entre elles d'au moins une cire solide et d'au moins une cire liquide en présence d'au moins un polyol et d'un catalyseur.

2. Procédé selon la revendication 1, ***caractérisé* en ce que** la cire solide est choisie dans le groupe comprenant la cire de carnauba, la cire de candelilla, la cire de son de riz, la cire de tournesol, la cire de canne à sucre, la cire d'ouricury, la cire d'abeille, la cire de Shellac.

3. Procédé selon la revendication 1, ***caractérisé* en ce que** la cire liquide est de la cire de jojoba.

4. Procédé selon la revendication 1, ***caractérisé* en ce que** le polyol est choisi dans le groupe comprenant l'éthylène glycol, le diéthylène glycol, le triéthylène glycol, le propylène glycol, le butylène glycol, le néopentyl glycol, le 2-méthyl propanediol, l'hexylène glycol, l'octylène glycol, le polyéthylène glycol, le polypropylène glycol, le triméthylol propane, le sorbitol, l'érythritol, le pentaerythritol, le dipentaerythritol, le glycérol, le diglycérol, et le polyglycérol.

5. Procédé selon la revendication 1, ***caractérisé* en ce que** la réaction de transestérification est réalisée entre la cire de jojoba, la cire de riz, la cire de candelilla et le polyglycérol.

6. Procédé selon l'une des revendications 1 à 5, ***caractérisé* en ce que** la réaction est conduite à une température comprise entre 100°C et 220°C.

7. Procédé selon l'une des revendications 1 à 6, ***caractérisé* en ce que** la réaction est conduite à une température comprise entre 150°C et 200°C.

8. Procédé selon la revendication 1, ***caractérisé* en ce que** le rapport massique cire liquide / cire solide varie entre 5/95 et 95/5.

9. Procédé selon la revendication 1, ***caractérisé* en ce que** le rapport massique cire liquide / cire solide varie entre 30/70 et 75/25.

10. Procédé selon la revendication 1, ***caractérisé* en ce que** le rapport massique cires/polyol varie entre 95/5 et 50/50.

11. Procédé selon la revendication 1, ***caractérisé* en ce que** le catalyseur est choisi dans le groupe comprenant les hydroxydes ou alkoxydes alcalins ou alcalino terreux, les carbonates de potassium ou de sodium, les catalyseurs à base d'étain ou de titane.

12. Procédé selon la revendication 1, ***caractérisé* en ce que** le catalyseur est choisi dans le groupe comprenant la soude, la potasse, et l'hydroxyde de calcium.

## Patentansprüche

1. Verfahren zur Herstellung einer Stütze durch gleichzeitige Umesterung miteinander mindestens eines festen Wachses und mindestens eines flüssigen Wachses, in Anwesenheit mindestens eines Polyols und eines Katalysators.

2. Verfahren nach Anspruch 1, ***dadurch gekennzeichnet, dass*** das feste Wachs aus der Gruppe bestehend aus Carnauba - Wachs, Candelilla - Wachs, Reiskeim - Wachs, Sonnenblumen - Wachs, Zuckerrohr - Wachs, Ouricury - Wachs, Bienenwachs, Schellack - Wachs ausgewählt wird.

3. Verfahren gemäß Anspruch 1, ***dadurch gekennzeichnet, dass*** das flüssige Wachs Jojoba - Wachs ist.

4. Verfahren gemäß Anspruch 1, ***dadurch gekennzeichnet*, *dass*** das das Polyol aus der Gruppe umfassend Ethylenglycol, Diethylenglycol, Triethylenglycol, Propylenglycol, Butylenglycol, Neopentylglycol, 2 - methyl - propanediol, Hexylenglycol, Octylenglycol, Polyethylenglycol, Polypropylen - Glycol, Trimethylol - Propan, Sorbitol, Erythritol, Pentaerythritol, Dipentaerythritol, Glyzerin, Diglyzerin und Polyglyzerin ausgewählt wird.

5. Verfahren gemäß Anspruch 1, ***dadurch gekennzeichnet*, *dass*** die Umesterungsreaktion zwischen Jojoba - Wachs, Reiswachs, Candelilla - Wachs und Polyglyzerin ausgeführt wird.

6. Verfahren gemäß einem der Ansprüche 1 bis 5, ***dadurch gekennzeichnet*, *dass*** die Reaktion bei einer Temperatur zwischen 100°C und 220°C ausgeführt wird.

7. Verfahren gemäß einem der Ansprüche 1 bis 6, ***dadurch gekennzeichnet, dass*** die Reaktion bei einer Temperatur zwischen 150°C und 200°C ausgeführt wird.

8. Verfahren gemäß Anspruch 1, ***dadurch gekennzeichnet, dass*** das Gewichtsverhältnis flüssiges Wachs / festes Wachs zwischen 5/95 und 95/5 liegt.

9. Verfahren gemäß Anspruch 1, ***dadurch gekennzeichnet, dass*** das Gewichtsverhältnis flüssiges Wachs / festes Wachs zwischen 30/70 und 75/25 liegt.

10. Verfahren gemäß Anspruch 1, ***dadurch gekennzeichnet, dass*** das Gewichtsverhältnis Wachsen/ Polyol zwischen 95/5 und 50/50 liegt.

11. Verfahren gemäß Anspruch 1, ***dadurch gekennzeichnet, dass*** der Katalysator aus der Gruppe, zu der Alkali - oder Erdalkali - Hydroxide oder - Alkoxide, Kalium - oder Natriumkarbonate und Katalysatoren auf Zink - oder Titanbasis gehören, ausgewählt wird.

12. Verfahren gemäß Anspruch 1, ***dadurch gekennzeichnet, dass*** der Katalysator aus der Gruppe, zu der Natron, Pottasche und Kalziumhydroxid gehören, ausgewählt wird.

## Claims

1. Process for preparing an excipient by a simultaneous transesterification reaction between at least one solid wax and at least one liquid wax in presence of at least one polyol and one catalyst.

2. Process according to claim 1, **characterized in that** the solid wax is chosen in the group comprising carnauba wax, candelilla wax, rice bran wax, sunflower wax, sugarcane wax, ouricury wax, beeswax, Shellac wax.

3. Process according to claim 1, **characterized in that** the liquid wax is jojoba wax.

4. Process according to claim 1, **characterized in that** the polyol is chosen in the group comprising ethylene glycol, diethylene glycol, triethylene glycol, propylene glycol, butylene glycol, neopentyl glycol, 2-methyl propanediol, hexylene glycol, octylene glycol, polyethylene glycol, polypropylene glycol, trimethylolpropane, sorbitol, erythritol, pentaerythritol, dipentaerythritol, glycerol, diglycerol, and polyglycerol.

5. Process according to claim 1, **characterized in that** the transesterification reaction occurs between jojoba wax, rice wax, candelilla wax and polyglycerol. 1.

6. Process according to one of claims 1 to 5, **characterized in that** the reaction is conducted at a temperature comprised between 100°C and 220°C.

7. Process according to one of claims 1 to 6, **characterized in that** the reaction is conducted at a temperature comprised between 150°C and 200°C.

8. Process according to claim 1, **characterized in that** the weight ratio liquid wax/solid wax ranges from 5/95 to 95/5.

9. Process according to claim 1, **characterized in that** the weight ratio liquid wax/solid wax ranges from 30/70 to 75/25.

10. Process according to claim 1, **characterized in that** the weight ratio waxes/polyol ranges from 5/95 to 50/50

11. Process according to claim 1, **characterized in that** the catalyst is chosen in the group comprising hydroxides or alkoxides of alkali or alkaline earth, potassium or sodium carbonates, and catalysts based on tin or on titanium.

12. Process according to claim 1, **characterized in that** the catalyst is chosen in the group comprising caustic soda, caustic potash and calcium hydroxide.
